# EUROPEAN PATENT APPLICATION

(11) **EP 3 603 503 A1**
(43) Date of publication of application: **05.02.2020**
(21) Application number: 19197859.2
(22) Date of filing: 17.02.2017
(51) Int. Cl.: A61B 5/08, A61B 5/16

(54) **METHOD FOR AUTOMATED IMPROVING INDOOR AIR**

(30) Priority: 19.02.2016 EP 16156510
(62) Divisional of application: 17708185.8
(71) Applicant: Patonomics AB, 414 64 Göteborg (SE)
(72) Inventor: Mitra, Kalyan, 414 64 Gothenburg (SE)

(57) **Abstract**

Method and apparatus for automated real-time artificial addition and/or subtraction of unique molecules in indoor air that normally mammals emit as metabolic product(s) under the influence of mammal's real-time emotional status

## Description

### Field of the invention

The present invention generally relates to a method and apparatus for automated real-time artificial addition and/or subtraction of unique molecules in indoor air that normally mammals emit as metabolic product(s) under the influence of mammal's real-time emotional state.

### Background of the invention

This is a divisional patent of application that initiates from earlier main European patent application 17708185.8 - 1132.

Living mammals, such as humans but also many animals, have many transitory emotional states occurring from time to time, such as anger, fear, laughter, happiness, etc. Recognizing such transitory emotional states is of great importance in many situations, such as in evaluating the impact and effect of real-time emotional wellbeing in multiple situations like personal, professional and commercial settings, and ability of activating and offering targeted real time response mechanism matching with transitory emotional states.

Indoor air is contaminated with few emotion specific unique molecules that mammals emit as metabolic product(s) under the influence of mammal's real-time emotional state. We described them in our earlier main European patent application 17708185.8 - 1132, and will now here we regulate their indoor concentration in residential, commercial, official, hospitals, educational institutes and transportation environment (like space crafts, airplanes, rockets, boats, yacht, ship, tram, trucks, bus, cars , trams and all others) where mammals are involved to have mammal's better emotional state. In this patent application we do not claim any non-metabolic product(s) like oxygen, nitrogen etc.

### Summary of the invention

It is a general object of the present invention to alleviate the above-discussed problems, and at least partly satisfying the above-discussed needs.

This object is fulfilled by a method and an apparatus for identification of at least one transitory emotional states of a living mammal in accordance with the appended claims.

According to the present invention, there is provided a method for automated improvement of an indoor environment, comprising the steps:
mounting at least one sensor on the environment for identification of at least one molecular concentration in an indoor environment related to at least one transitory emotional state of living mammal(s);
determining the quantity of said molecular concentration in the indoor environment;
comparing the determined quantity of said molecular concentration in the indoor environment to a predetermined threshold value;
artificially adding or subtracting the said molecular concentration in an indoor environment.

By means of this aspect, the indoor environment can automatically be improved, so as to make the indoor environment more emotionally motivating or attractive for the emotional state of the living mammal(s). For example, this may be used to add positive emotion promoting molecule(s) in real time, or to subtract negative emotion promoting molecule(s) in real time.

These and other aspects of the inventive concept will be apparent from and elicited with reference to the embodiments described hereinafter.

The identified transitory emotional state may be used in at least one of:
- Injecting right quantity positive emotional and motivational volatile biomarkers and/or expelling or destroying the negative emotional and motivational volatile biomarkers may be used for many other purposes as well, e.g. to increase the efficiency of psycho-therapy sessions.
- Aid in commercial and/or residential environments. It may also be useful for improving and customizing consumer environment by injecting right quantity positive emotional and motivational volatile biomarkers and/or expelling or destroying the negative emotional and motivational volatile biomarkers from the closed or close-like environments at different kind commercial spaces, like, school, shopping places, shopping malls, hospitals, offices, movie theaters, stadiums, etc. It may also be used to improve the indoor environment of a residence.
- Aid in transportation environments. It may also be useful for improving and customizing transportation environment by injecting right quantity positive emotional and motivational volatile biomarkers and/or expelling or destroying the negative emotional and motivational volatile biomarkers from the closed or close-like environments at different including all transportation and spacecrafts environments.

### Description of preferred embodiments

In the following detailed description preferred embodiments of the invention will be described. However, it is to be understood that features of the different embodiments are exchangeable between the embodiments and may be combined in different ways, unless anything else is specifically indicated. Further, even though the following detailed description focuses on monitoring of human beings, it is to be appreciated by the skilled reader that it is equally applicable to animals and in particular commercial and/or domestic animals or zoo animals, such as horses, dogs, cows, cats, primates, rats, mice and the like. It is noticed that commercial and domesticated animals smell out emotions in other living mammals and react accordingly.

A sensor may be configured to measure one or several metabolite molecules emanating from the breath or through the skin. Further, measurement methods for determining presence and quantity of various metabolite molecules are also per se known. For example, such measurement may involve Proton Transfer Reaction Mass Spectrometry (PTR-MS), Selected Ion Flow Tube Mass Spectrometry (SIFT-MS) and Ion Mobility Spectrometry (IMS). These and other suitable measurement methods are per se known, and are e.g. further discussed in "Dynamic profiles of volatile organic compounds in exhaled breath as determined by a coupled PTR-MS/GC-MS study" by J. King et al, Physiological Measurement, No. 31 (2010), 1169-1184, "Ammonia in breath and emitted from skin" by F. Schmidt et al, Journal of Breath Research, No. 7 (2013), and "Breath Analysis as a Potential and Non-Invasive Frontier in Disease Diagnosis: An Overview" by J. Pereira et al, Metabolites, No. 5, 2014, all of said documents hereby being incorporated in their entirety by reference. There are multiple research labs and companies that offer mico and nano sensors that have capability to measure specific gasses or volatile compounds in real time or near real time. The sensor for wearable emotion sensing may preferably use portable gas sensors. Such sensors are e.g. commercially available from Sensirion and Sensotran, and there are also known gas sensors technologies that use infrared or near infrared for real time gas sensing. There is at present a lot of research related to miniaturization of real time gas sensors, and such gas sensors, when commercially available, can also be used for implementing the invention.

The sensor may be arranged as a separate part, and is preferably communicating with the analyzer over a wireless connection, such as by Bluetooth. However, the sensor may also have a wired connection to the analyzer, or even form an integrated unit together with the analyzer.

The analyzer is a computer/processor-controlled part, arranged to perform the above-discussed method for identification of transitory emotional state(s) by executing a software code. However, the method may also be partly or fully implemented by hardware.

The analyzer is configured to identify one or several transitory emotional state(s) by executing the following steps:
- Reception of measurement data from the sensor or from the memory
- Analyzing the measurement data related to the amount of the at least one metabolite molecule(s) to establish whether the amount or the increase rate of said at least one metabolite molecule(s) exceeds at least one predetermined criterion;
- Determining, when such a predetermined criterion has been exceeded, that a transitory emotional state associated with this at least one metabolite molecule(s) is present in the living mammal; and
- Forwarding, optionally, the result of the analysis and/or the determination to the output device and/or to the memory for storage.

The memory may be any type of memory device arranged to store data in a retrievable fashion.

The output device may take various forms. It may be a display, for presenting information in relation to the determined emotional/mental state(s), in writing, by showing images, showing concentration pots over time, or the like. It may also be one or several lights, e.g. lights of various colors, which are illuminated, upon identification of a particular emotional/mental state. For example, a green light may be active when happiness has been detected, a red light may be active when fear or anger has been detected, and a yellow light may be active when no emotional state has been identified. The output device may also be a loudspeaker, emitting signals indicative of the identified mental state(s). Many other type of output devices may also be used, as would be apparent for the skilled reader. The aggregate review of emotions can be visualized and viewed for any time period, such as specific seconds, specific hours, specific days, weeks, months, year or years. Various combinations of these and other output devices may also be used.

Data regarding e.g. identified emotional/mental state(s), measurement data, etc may also be forwarded to separate external devices, such as to an external tablet, general purpose computer, electronic watch, smart phone, etc. This transfer of data may be provided through wired connections, but preferably occurs through wireless communication, such as by conventional TCP/IP communication. To this end, both the analyzer and the separate external device may preferably be provided with capability and access to communicate via the Internet.

The analyzer may also send an alarm signal or the like to an external device when certain mental state(s) has been identified. For example, an alarm may be sent to caregiver or nursing personnel when a patient experiences fear.

In such a case, when issuing an alarm, the apparatus may further communicate the present position of the apparatus together with the alarm. Such position data may be entered manually to the apparatus, or be retrieved from other sources. However, the apparatus may also comprise a Global Navigation Satellite System (GNSS) receiver, such as a GPS receiver, to obtain such positioning data.

The apparatus may further comprise an input device (not shown), such as a keyboard or the like, enabling the user to modify the performance of the device. For example, this may be used to vary the identification criteria used by the device, so that the sensitivity of the apparatus is controlled to a desired level. It may also be used to control the output format, and the like. Additionally, or alternatively, such control of the apparatus may also be obtained through one or several external devices, the external device(s) thereby functioning as a remote control for the apparatus.

Then artificially adding or subtracting the said molecular concentration in an indoor environment using known molecule injecting and subtracting device.

According to the present invention, there is provided a method for automated improvement of an indoor environment, comprising the steps:
mounting at least one sensor on the environment for identification of at least one molecular concentration in an indoor environment related to at least one transitory emotional state of living mammal(s);
determining the quantity of said molecular concentration in the indoor environment;
comparing the determined quantity of said molecular concentration in the indoor environment to a predetermined threshold value;
artificially adding or subtracting the said molecular concentration in an indoor environment.

By means of this aspect, the indoor environment can automatically be improved, so as to make the indoor environment more emotionally motivating or attractive for the emotional state of the living mammal(s). For example, this may be used to add positive emotion promoting molecule(s) in real time, or to subtract negative emotion promoting molecule(s) in real time.

## Claims

1. A method for automated improvement of an indoor environment, comprising the steps:
mounting at least one sensor on the environment for identification of at least one molecular concentration in an indoor environment related to at least one transitory emotional state of living mammal(s);
determining the quantity of said molecular concentration in the indoor environment;
comparing the determined quantity of said molecular concentration in the indoor environment to a predetermined threshold value; and
artificially adding or subtracting the said molecular concentration in an indoor environment.

2. The method of claim 1, wherein the step of artificially adding or subtracting comprises at least one of adding positive emotion promoting molecule(s) in real time, and subtracting negative emotion promoting molecule(s) in real time.
